# EUROPEAN PATENT APPLICATION

(11) **EP 2 801 566 A1**
(43) Date of publication of application: **12.11.2014**
(21) Application number: 14162529.3
(22) Date of filing: 31.03.2014
(51) Int. Cl.: C07D 209/02

(54) **Process for the preparation of a viral protease inhibitor and its intermediates**

(30) Priority: 30.04.2013 IT MI20130706
(71) Applicant: Dipharma Francis S.r.l., 20021 Baranzate (MI) (IT)
(72) Inventor: Iuliano, Anna, 20021 BARANZATE (MI) (IT); Jumde, Varsha Ravindra, 20021 BARANZATE (MI) (IT); Attolino, Emanuele, 20021 BARANZATE (MI) (IT); Taddei, Maurizio, 20021 BARANZATE (MI) (IT)
(74) Representative: Bertuccio, Silvia

(57) **Abstract**

Process for the preparation of a viral protease inhibitor and intermediates useful in its preparation.

## Description

### FIELD OF INVENTION

The present invention relates to a process for the preparation of a viral protease inhibitor and intermediates useful for its preparation.

### PRIOR ART

(1S, 3aR, 6aS)-2-[(2S)-2-[[(2S)-2-cyclohexyl-2-[(2-pyrazinylcarbonyl) amino]acetyl]amino-3,3-dimethylbutanoyl]-N-[(1S)-1-[(cyclopropylamino) (oxo)acetyl]butyl]-3,3a,4,5,6,6a-hexahydro-1H-cyclopenta[c]pyrrole-3-carboxamide of formula **(I),** also known as telaprevir, is a potent viral protease inhibitor used to treat hepatitis C infections.

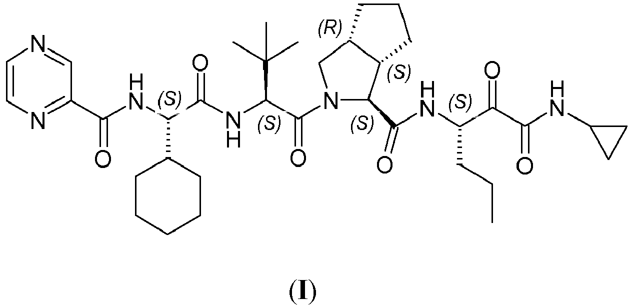

The preparation of telaprevir, reported in US 7820671, involves the assembly of 6 different structural units to create 5 amide bonds (Scheme 1) 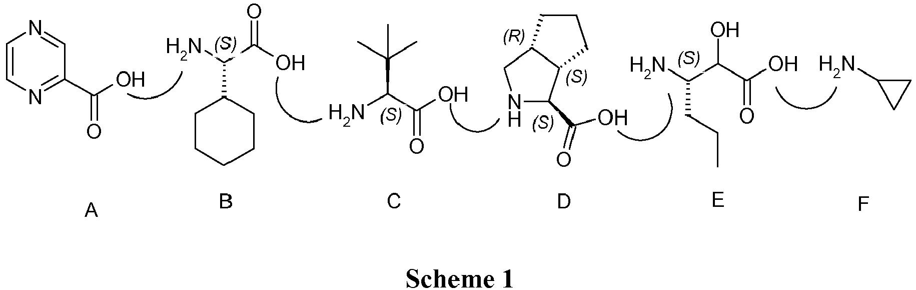 and subsequent oxidation of the alcoholic hydroxyl group of the compound of formula E. The compounds of formulas A and F are pyrazine-carboxylic acid and cyclopropylamine respectively.

The compounds of formulas B, C, D and E are four amino acids, all with the (S) configuration.

The compounds of formulas B and C are (S)-cyclohexylglycine and (S)-tert-leucine respectively, namely simple amino acids available on the market, while the compounds of formulas D and E are two synthetic amino acids with a more complex structure.

The preparation of the compounds of formulas D and E is reported in US 7820671.

While the preparation of the amino acid derived from norvaline of formula E has long been reported in the literature and can be performed by known methods, the preparation of the key amino acid of formula D is rather complex, and uses expensive reagents which are often not commercially available, especially if the expensive chiral phase-transfer catalysts (chiral PTC) are used.

More recently, US 7776887 reported the synthesis of the bicyclic amino acid of formula D, involving the introduction of the carboxyl group into the protected amino derivative of formula G by formation of the organolithium intermediate of the protected amino compound of formula G, obtained by reaction with lithium diisopropylamide (LDA), and its subsequent carboxylation, according to Scheme 2 below.

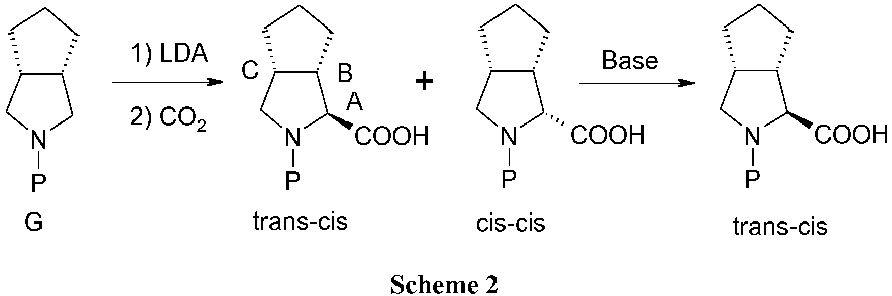

The mixture of diastereoisomers of the carboxyl intermediate thus formed, due to the formation of three *trans-cis, cis-cis* stereocenters in the A, B and C positions, is then subjected to partial epimerisation of stereocentre A to give only the desired racemic *trans-cis* isomer, which is then subjected to resolution via diastereomeric salts to give the desired resolved amino acid compound of formula D.

In this case too, although the process involves the use of a cheap raw material, the specific reaction conditions, such as functionalisation with the use of organometals at low temperature, unfortunately prevent its use on an industrial scale.

Recently in Angew. Chem. Int. Ed. 2010, 49, 2182-2184 and WO 2010/008828, starting with the amino derivative of formula G, they were able to introduce the carboxyl functionality without the use of organometallic reactions. In fact, 3-azabicyclo[3.3.0]octane is converted to the corresponding optically active imine by a complex biocatalytic process of oxidative desymmetrisation with the use of monoamine oxidase enzymes (MAO), Scheme 3. The addition of cyanide to the double imine bond, obtained by using alkaline metal cyanides or trimethylsilyl cyanide, after hydrolysis of the nitrile functionality to carboxylic acid, gives rise to the desired amino acid with the corresponding trans-cis configuration with enantiomeric excesses of 98%.

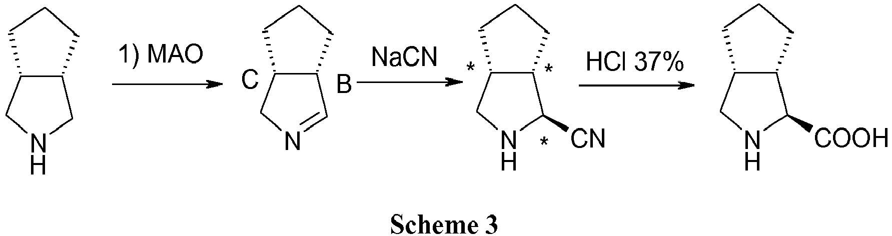

In this case, the formation of the optically active imine with the biochemical system and the use of cyanides makes the process fairly short but complex, not very productive, and above all dangerous.

There is consequently a need for a more advantageous alternative method for the preparation of telaprevir, and its synthetic intermediates, on an industrial scale. Said novel method should in particular be more industrially scalable, involve the use of cheaper reagents which are more easily handled and safer for humans and the environment, with mild reaction conditions, and at the same time provide high yields of the desired compounds.

### SUMMARY OF THE INVENTION

The subject of the present invention is a process for the preparation of a compound of formula **(II),** as a single enantiomer or a racemic mixture or salt thereof 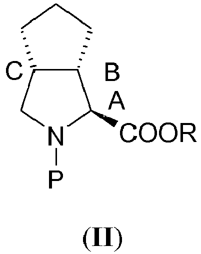 wherein P and R are as defined below and A, B and C on the azabicyclo indicate three stereocenters, comprising oxidation of a compound of formula **(III)** as a stereoisomeric mixture or a single stereoisomer 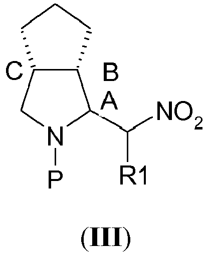 to give a compound of formula **(II),** and the possible chemical or enzymatic resolution of the compound of formula **(II)** thus obtained.

### DETAILED DESCRIPTION OF THE INVENTION

The invention relates to a process for the preparation of a compound of formula **(II),** as a racemic mixture or a single stereoisomer, or a salt thereof, 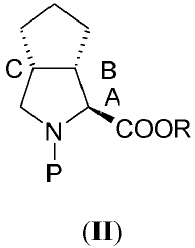 wherein R is H or an optionally substituted C₁-C₁₂ alkyl group, an optionally substituted C₃-C₁₀ cycloalkyl group, or an optionally substituted aryl or heteroaryl group; P is H or an amino protecting group, comprising oxidation of a compound of formula **(III)** as a stereoisomeric mixture or a single stereoisomer 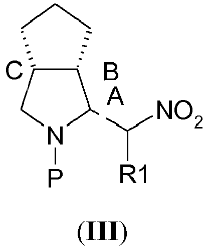 wherein P is as defined above and R1 is H or methyl and, if desired, the conversion of a thus obtained compound of formula **(II)** to another compound of formula **(II)** and/or, if desired, the conversion of a compound of formula **(II)** to a salt thereof and/or, if desired, the conversion of a salt of a compound of formula **(II)** to the free acid.

A salt of a compound of formula **(II)** is typically a pharmaceutically acceptable salt.

A C₁-C₁₂ alkyl group, which may be straight or branched, is, for example, a C₁-C₄ alkyl group optionally substituted by one to three substituents such as halogen, typically fluorine, and preferably tert-butyl.

A C₃-C₁₀ cycloalkyl group, which can be optionally substituted, for example, by one to three halogen atoms, is preferably a C₃-C₇ cycloalkyl group, in particular cyclopropyl and cyclohexyl group.

An aryl group is, for example, phenyl, naphthyl, optionally substituted, for example, by one to three atoms of halogen, typically fluorine; preferably phenyl.

A heteroaryl group can be heteromonocyclic or heterobicyclic, unsaturated, saturated or partly unsaturated, containing one to three heteroatoms independently selected from oxygen, nitrogen and sulphur, and optionally substituted, for example, by one to three halogen atoms, typically fluorine. The preferred heteroaryls are pyridyl and imidazolyl.

An amino protecting group can be, for example, a protecting group known from peptide chemistry, preferably tert-butyloxycarbonyl (Boc) or benzyloxycarbonyl (Cbz) group.

The stereocenters in the B and C position in a compound of formula **(III)** present on the junction of the bicyclic system have the (S) and (R) configuration respectively, so that the two bonds present on the cyclopentane residue are in *cis* to one another, namely on the same side of the cycle, whereas the absolute configuration of the stereocentre in the A position marked on the ring can be (R) or (S), preferably (S).

When R1 is H and P is as defined above, a compound of formula **(III)** can be converted to a compound of formula **(II),** as defined above, by a process comprising:
i) reacting a compound of formula **(III)** with an oxidising agent, possibly in a solvent; or
ii) converting a compound of formula **(III)** to a compound of formula **(IV)** 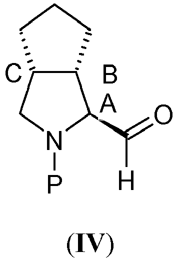
wherein P is as previously defined, and its subsequent conversion to a compound of formula **(II),** as defined above.

An oxidising agent, according to variant i) of the process as defined above, can typically be an oxidant selected from the group comprising potassium permanganate, sodium nitrite, potassium peroxymonosulphate, a mixture of potassium hydrogen sulphate and potassium peroxydisulphate such as the mixture commercially known as Oxone®, and molecular oxygen in the presence of a copper-based catalyst.

Said oxidising agent is preferably sodium nitrite or potassium permanganate.

A solvent according to variant i) of the process as defined above can be a solvent miscible or immiscible with water, and oxidation can be performed in a monophasic or biphasic system, the latter consisting, for example, of an aqueous solution of the oxidising agent as defined above and an organic solvent immiscible with water. An organic solvent can be a polar aprotic solvent, typically dimethylformamide, dimethylacetamide, N-methylpyrrolidone, acetonitrile or dimethyl sulphoxide; a cyclic or acyclic ether such as tetrahydrofuran, dioxane, a chlorinated aliphatic or aromatic solvent such as dichloromethane, dicloroethane, chloroform or chlorobenzene; an ester, such as ethyl or methyl acetate; a polar protic solvent such as a straight or branched C₁-C₈ alkanol, for example a C₁-C₅ alkanol, typically methanol, ethanol, n-propanol, isopropanol, n-butanol, isobutanol, tert-butanol or water, or a buffered aqueous solution, such as an aqueous solution of phosphate buffer or a mixture of two or more, preferably two or three, of said solvents.

The reaction according to variant i) as defined above can be performed at a temperature ranging between about 0°C and the reflux temperature of the solvent, preferably between about 10°C and 40°C, and more preferably at room temperature.

According to variant ii), a compound of formula **(III),** wherein R1 is H, can typically be converted to a compound **(IV)** by a process comprising:
a) treating a compound of formula **(III)** with an oxidising agent selected, for example, from the group comprising hydrogen peroxide, an organic peroxide or a buffered solution of potassium permanganate; or
b) treating a compound of formula **(III)** with an oxidising agent used in the process described in variant i), as defined above, but under mild reaction conditions; or
c) treating a compound of formula **(III)** under Nef reaction conditions, wherein the compound of formula **(III),** in a strongly basic reaction medium, is converted to an intermediate nitronate salt which, in turn, under strongly acid reaction conditions, is subsequently converted to the compound of formula **(IV),** as defined above.

According to variant ii), the aldehyde compound of formula **(IV)** thus obtained can be converted to a carboxyl compound of formula **(II)** wherein R is H and P is as defined above, according to known methods. Said conversion can, for example, be performed by treating the compound of formula **(IV)** with AgNO₃, Jones reagent and the TEMPO/chlorite system.

Instead, in a compound of formula **(III),** wherein R1 is methyl and P is as previously defined, oxidation of a compound of formula **(III)** to a compound of formula **(II)** can be performed by a process comprising the conversion of a compound of formula **(III)** to a compound of formula **(V)** 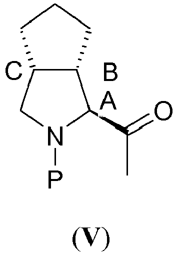 wherein P is as defined above, and the subsequent conversion of said compound of formula **(V)** to the compound of formula **(II),** as defined above.

In particular, in this case, the conversion of the compound of formula **(III),** wherein R1 is methyl and P is as defined above, can be performed by one of the known methods for conversion of secondary nitro alkyls to a carbonyl group, for example in the presence of an oxidising agent as defined above, optionally in a solvent as defined above, or under the hydrolysis conditions of the Nef reaction as reported above.

Thus the subsequent conversion of a compound of formula **(V),** as defined above, to the compound of formula **(II),** wherein R is H and P is as defined above, can be performed by one of the methods for transformation of methyl ketones to the known carboxylic acids, for example by haloform reaction.

The haloform reaction can be performed, for example, by treating a compound of formula **(V)** with a halogen, preferably bromine or iodine, in a basic aqueous environment, or by treating it with a compound able to halogenate methyl ketones, for example with sodium hypochlorite or N-bromosuccinimide.

The compounds of formulas **(III), (IV)** wherein P being as defined above is not terbutyloxcarbonyl (Boc), and **(V)** are novel, and represent a further subject of the invention.

A compound of formula **(II),** prepared as reported above, starting with a compound of formula **(III)** wherein R1 is H, or with a compound of formula **(III)** wherein R1 is methyl and P is as defined above, presents the free carboxyl functionality, wherein R is equal to H and P is as defined above.

The conversion of a compound of formula **(II),** wherein R is H and P is as defined above, to a compound of formula **(II)** wherein R, being as defined above, is different from H, and P is as defined above, can be performed by esterification reaction according to known methods.

Conversely, a compound of formula **(II),** wherein R, being as defined above, is different from H, and P is as defined above, can be converted to a compound of formula **(II),** wherein R is H and P is as defined above, according to known methods, for example by treatment in a strongly basic, aqueous or anhydrous environment, for example by treatment with an aqueous solution of sodium hydroxide or a solution of potassium tert-butoxide in an organic solvent.

A compound of formula **(II)** wherein P is a protecting group can be converted to a compound of formula **(II)** wherein P is hydrogen, by known methods. Similarly, a compound of formula **(II)** wherein P is H can be converted to a compound of formula **(II)** wherein P is a protecting group, by known methods.

A thus obtained compound of formula **(II)** can be converted to a salt thereof, and similarly, a salt thereof can be converted to a free compound of formula **(II);** said conversions can be performed by known methods.

Independently of the stereochemistry of the stereocentre in the A position of a compound of formula **(III),** the conditions of the oxidation reaction or treatment in a basic environment of the ester group in a compound of formula **(III)** are such that even starting with a compound of formula **(III)** wherein the relative stereochemistry between stereocenters A, B and C is cis-cis, a compound of formula **(II)** is obtained wherein the relative stereochemistry is trans-cis. This is because, according to the process according to the invention, the stereocentre is epimerised in the A position of the optional cis-cis diastereoisomer to the more stable trans-cis diastereoisomer.

A compound of formula **(II),** wherein R and P are as defined above, prepared as described so far, presents a relative trans-cis configuration of stereocenters A, B and C, and is a racemate.

A compound of formula **(II)** in optically active form, wherein R and P are as defined above, can be prepared by resolution of its corresponding racemic mixture, prepared as described above, by a process comprising enantioselective enzymatic hydrolysis of the ester group of an enantiomeric compound of formula **(II)** wherein R, being as defined above, is different from H, and P is as defined above, being present in said racemic mixture in the presence of an enzyme, in a solvent mixture. The enantioselective enzymatic hydrolysis reaction can be performed with an enzyme belonging to the protease or lipase group. Said enzyme can derive from various sources such as bacteria, fungi, animals or plants.

A lipase or a protease, according to the invention, preferably belongs to the class of those active at a pH of between about 6 and 9.

The enantiomers of a compound of formula **(II),** or a salt thereof, can preferably be separated with a lipase. In particular, enantioselective enzymatic hydrolysis of a compound of formula **(II)** can be advantageously performed with high yields and chemical and stereochemical purity, evaluated by HPLC, using a lipase deriving from *Aspergillus niger.*

A solvent mixture is formed, for example, by a solution comprising an aqueous buffer at a pH of between about 6.0 and 9.0, preferably around a pH of about 7.5, and optionally an organic co-solvent, miscible or immiscible with the buffer.

A solution of an aqueous buffer may, for example, be a known phosphate buffer, ammonium bicarbonate, ethanolamine/HCl or borate; the reaction is preferably performed in phosphate buffer.

An organic co-solvent may, for example, be a polar aprotic solvent such as dimethylformamide, dimethylacetamide, acetonitrile or dimethyl sulphoxide; a ketone, such as acetone or methyl isobutyl ketone; an ether, such as tetrahydrofuran or dioxane; or an apolar aprotic solvent, preferably an apolar aprotic solvent in particular toluene.

The hydrolysis reaction can be performed at a temperature of between about 15 and 60°C, preferably between about 20 and 40°C, and more preferably at about 25°C. The reaction times depend on the reaction temperature and the type of enzyme used. In this way, one of the two enantiomers of a compound of formula **(II),** which is not a substrate for the enzyme, remains unchanged as ester, while the other enantiomer, which is a substrate for the enzyme, is hydrolysed to obtain a compound of formula **(II)** wherein R is a free carboxyl group, and consequently as free carboxylic acid.

The optically pure non-hydrolysed enantiomer of a compound of formula **(II),** wherein R, being as defined above, is different from H, and P is a protecting group as defined above, which is consequently still present as carboxylic ester in the end-of-reaction mixture, can be isolated from the reaction mixture by extraction with an organic solvent immiscible with water, such as ethyl acetate, optionally after removal of protecting group P of the amino group, if any.

Conversely, when the organic phase has been separated from the aqueous phase, the hydrolysed enantiomer of formula **(II),** wherein R is H and P is as defined above, can be recovered by acidifying the saline end-of-reaction mixture, wherein the carboxylic acid is in salified form, to a pH of about 4-5 by adding an acid, such as hydrochloric acid, and extracting with a solvent immiscible with water, such as ethyl acetate.

By concentrating the organic solution, the enantiomer of a compound of formula **(II)** is obtained, wherein R is H and P is a protecting group as defined above, namely free carboxylic acid.

Alternatively, in the case of enantioselective enzymatic hydrolysis of a compound of formula **(II)** wherein P is H and R, being as defined above, is different from H, the extraction in organic phase of the compound of formula **(II),** wherein R is different from H and P is H, can be performed after converting said compound of formula **(II)** wherein P is H to a compound of formula **(II)** wherein P is a protecting group.

Both enantiomers of a compound of formula **(II),** wherein R and P are as defined above, are thus obtained with excellent yields, typically between about 40 and about 50%, starting from the racemate of formula **(II),** and a chemical purity evaluated by HPLC as equal to or greater than 95%, preferably equal to or greater than 98%.

The enantiomeric purity of the isolated enantiomers of formula **(II),** calculated by HPLC on chiral stationary phase, is expressed in terms of enantiomeric ratio, and is typically equal to or greater than 98:2, and preferably equal to or greater than 99:1.

If the case, a single enantiomer of a compound of formula **(II),** thus obtained, can be converted to another enantiomeric compound of formula **(II)** having the same configuration, according to known methods.

For example, an enantiomer of a compound of formula **(II),** wherein R is H, can be converted to a salt thereof by reaction with an organic or inorganic base, preferably a tertiary amine, in a solvent, according to known methods.

A further purpose of the present invention is therefore a process for the preparation of an enantiomerically pure compound of formula **(II),** or a salt thereof, comprising the preparation of a compound of formula **(II)** in racemic form by the process according to the invention, and its subsequent resolution by enantioselective enzymatic hydrolysis according to the process described above.

Alternatively, a racemic compound of formula **(II),** wherein P is a protecting group as defined above and R is H, can also be converted to an enantiomerically pure compound of formula **(II)** by the classic technique of resolution via diastereomeric salts obtained by reacting the racemic mixture of a compound of formula **(II)** with an optically active chiral amine, preferably (S)-1,2,3,4-tetrahydro-1-naphthylamine or (S)- phenylethylamine, as reported in WO 2007/022459, or (S)- naphthylethylamine.

A further purpose of the present invention is therefore a process for the preparation of an enantiomerically pure compound of formula **(II),** or a salt thereof, comprising the preparation of a compound of formula **(II)** in racemic form by the process reported above, and its subsequent resolution by formation of its diastereomeric salts obtained by reaction with a chiral amine.

An enantiomerically pure compound of formula **(II)** with a trans-cis relative configuration, and with the carbon stereocentre in position A with the (S) configuration, can be used in a process for the preparation of telaprevir of formula **(I)** according to known methods, such as those reported in US 7,820,671.

The invention therefore also relates to a process for the preparation of a compound of formula **(I)** comprising the use of a compound of formula **(II),** as a racemic mixture or in enantiomerically defined form, or a salt thereof, obtained by the process according to the present invention.

A compound of formula **(III),** wherein P is a protecting group as defined above and R1 is as defined above, can be prepared by a process comprising the addition of a compound of formula **(VI)**

R1CH₂NO₂ **(VI)**

wherein R1 is as defined above, to a racemic compound of formula **(VII)** 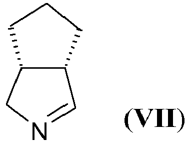 to obtain a compound of formula **(III)** 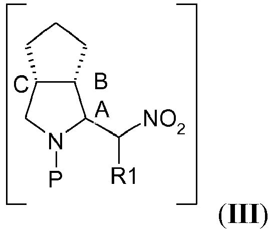 wherein R1 is as defined above and P is equal to H, and subsequent protection of the amino group in said compound of formula **(III).**

A further subject of the present invention is therefore a process for the preparation of a compound of formula **(III)** as defined above, according to the process described above.

The addition reaction can be performed by reacting a compound of formula **(VII)** with a compound of formula **(VI)** in the presence of a base and, if desired, of a solvent, to obtain the intermediate of formula **(III)** reported above. The subsequent protection of the amino functionality in said compound of formula **(III)** can be performed by methods well known to the skilled person.

A compound of formula **(VI)** can be used in a stoichiometric ratio with the compound of formula **(VII),** or used in excess as co-solvent of the reaction. It is preferably used in excess.

A base used in the addition reaction can be, for example, organic or inorganic. An organic base is typically a tertiary amine such as triethylamine, diisopropylethylamine or diazabicycloundecene (DBU). An inorganic base can be a hydroxide or a carbonate of an alkali or alkaline-earth metal, typically potassium carbonate.

A solvent used in the addition reaction can be, for example, a polar aprotic solvent such as dimethylformamide, dimethylacetamide, acetonitrile or dimethyl sulphoxide; a ketone, such as acetone or methyl isobutyl ketone; a cyclic or acyclic ether, such as tetrahydrofuran or methyl tert-butyl ether; an apolar aprotic solvent such as toluene, or a polar protic solvent such as a straight or branched C₁-C₅ alkanol, preferably methanol or isopropanol.

The reaction can be performed at a temperature of between about 15 and 60°C, preferably between about 20 and 40°C, and more preferably at about 25°C.

The compound of formula **(VI)** is commercially available.

The compound of formula **(VII)** can be prepared by oxidation of the 3-azabicyclo[3.3.0]octane of formula **(VIII),** 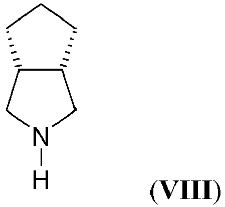 which is commercially available as the hydrochloride salt, by different methods, such as electrochemical anodic oxidation, treatment with an oxidising agent such as Na₂S₂O₈, as reported in Angew. Chem. Int. Ed. 2010, 49, 2182-2184, or by oxidation with N-chlorosuccinimide to give the corresponding N-chloro amine of formula **(IX)** 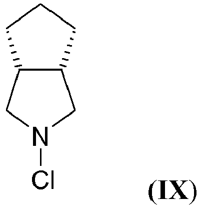 which, after treatment with an organic base, as defined above, leads to the formation of the compound of formula **(VII),** as defined above.

The following examples illustrate the invention.

### Example 1: Synthesis of a compound of formula (VII)

NaOH in flakes (0.54 g, 13.6 mmol) and AgN03 (7 mg, 0.04 mmol) were added to a solution of 3-azabicyclo[3.3.0]octane hydrochloride acid (1.0 g, 6.8 mmol) in 6.4 ml of H₂O. The mixture was cooled to 0°C, and then treated by slow dripping with a 25% solution of Na₂S₂O₈ (6.5 ml). After the addition, the reaction mixture was maintained under stirring at room temperature for 4 hours, and then treated with a saturated solution of NaCl and extracted with CH₂Cl₂ (3x25 ml). The combined organic phases were dried on anhydrous Na₂SO₄, filtered and concentrated at low pressure. The residue obtained was crystallised from methanol to obtain the compound of formula **(VII)** (0.35 g, 3.2 mmol) in the form of a chemically pure trimer, with a yield of 47%. However, NMR analysis in CDCl₃ shows the signals of both monomeric and trimeric species.

¹H NMR assigned to the trimer (200 MHz, CDCl₃, 25°C, δ): 3.21 (t, 3H, J 10Hz); 2.59-2.79 (m, 3H); 2.22-2.54 (m, 6H); 1.90 (dd, 3H, J₁=8 Hz, J₂=2 Hz); 1.78-1.17 (m, 18H).

¹H NMR assigned to the monomer (200 MHz, CDCl₃, 25°C, δ): 7.32 (m, 1H); 4.08 (dddd, J₁=18 Hz, J₂=10 Hz, J₃=4 Hz, J₁=2 Hz, 1H); 3.62-3.47 (m, 1H); 3.29 (m, 1H); 2.68 (m, 1H); 1.78-1.17 (m, 6H).

¹³C NMR assigned to the trimer (50 MHz, CDCl₃, 25°C, δ): 89.0; 54.1; 46.7; 41.1; 32.3; 30.9; 26.5.

¹³C NMR assigned to the monomer (50 MHz, CDCl₃, 25°C, δ):169.8; 70.2; 55.5; 39.0; 35.1; 29.7; 25.1.

### Example 2: Synthesis of a compound of formula (III), wherein R1 is H and P is Boc

A compound of formula **(VII),** obtained as in Example 1 (0.35 g, 3.2 mmol), is dissolved in anhydrous nitromethane (18 ml), and the resulting solution is treated with anhydrous triethylamine (3.2 mmol, 0.5 ml) under stirring. The reaction mixture is then maintained under stirring at room temperature for 16 hours, and treated with di-tert-butyl dicarbonate (1.4 g, 6.4 mmol). The reaction mixture is left under stirring for a further 3 hours at room temperature, then treated with water and extracted with Et₂O (4x15 ml). The combined organic phases are washed sequentially with 10% HCl, H₂O, 5% NaHCO₃ and H₂O, then dried on anhydrous Na₂SO₄, filtered and concentrated at low pressure. A residue is obtained which, when analysed by NMR, consists of a mixture of the compound of formula **(III)** and unreacted di-tert-butyl dicarbonate. A yield of 61 % was calculated on the basis of the integrals of the NMR signals. The crude product was used "as is" in the subsequent reaction; only a small portion was purified by flash chromatography for characterisation.

¹H NMR (200 MHz, CDCl₃, 50°C, δ): 4.64 (poorly resolved dd, 1H); 4.42 (broad t, 1H); 4.23 (poorly resolved ddd, 1H); 3.51 (dd, J₁=12, J₂=4, 1H); 3.29 (poorly resolved dd, 1H); 2.68 (m, 1H), 2.57 (m, 1H); 1.48 (s, 9H); 2.06-1.41 (m, 6H).

### Example 3: Synthesis of racemic hexahydro-cyclopenta[c]pyrrole-1,2-dicarboxylic acid 2-tert-butyl ester of formula (II), wherein R is H and P is Boc

The crude product of the reaction described in Example 2 is dissolved in DMSO (7 ml); NaNO₂ (0.72 g, 13 mmol) and acetic acid (2 ml) are added to the solution, and the mixture is maintained under stirring at 35°C for 6 hours. The reaction mixture is cooled to room temperature, treated with 10% HCl until it reaches pH 2-3, and then extracted with Et₂O (5x10 ml). The ether solution is concentrated to about 20 ml, and then extracted with 5% NaOH (4x7ml). The basic aqueous phase is washed with Et₂O (3x7 ml), and then acidified with 10% HCl (pH 2-3) and extracted with Et₂O (5x10 ml); finally, the combined ether extracts are dried on anhydrous Na₂SO₄, filtered and concentrated at low pressure. A residue consisting solely of a compound of formula **(II)** (0.45 g, 1.76 mmol) is obtained, with a yield of 91 %.

¹H NMR (200 MHz, CDCl₃, 50°C, δ): 7.23 (s sl, 1H); 4.12 (s sl, 1H); 3.68 (poorly resolved t, 1H); 3.30 (poorly resolved dd, 1H); 2.78 (m, 2H); 1.48 (s, 9H); 2.10-1.40 (m, 6H).

¹³C NMR (50 MHz, CDCl₃, 50°C, δ): 177.7, 154.8, 80.5, 65.8, 53.0, 49.4, 42.1, 33.0, 32.4, 28.5, 25.6.

### Example 4: Crystallisation of racemic hexahydro-cyclopenta[c]pyrrole-1,2-dicarboxylic acid 2-tert-butyl ester of formula (II), wherein R is H and P is Boc.

Heptane (150 mL) is added to hexahydro-cyclopenta[c]pyrrole-1,2-dicarboxylic acid 2-tert-butyl ester **(II)** (29.0 g, 113.7 mmol) obtained as in Example 3, and the mixture is left under stirring for 60 hours. The mixture is filtered and the solid washed with heptane to give the compound hexahydro-cyclopenta[c]pyrrole-1,2-dicarboxylic acid 2-tert-butyl ester **(II)** (23.0 g, 80%) in chemically pure form as a white crystalline solid.

Melting point: 88.6°C.

### Example 5: Preparation of (S)-1,2,3,4-tetrahydro-1-naphthylammonium (1S,3aR,6aS) hexahydro-cyclopenta[c]pyrrole-1,2-dicarboxylate 2-tert-butyl ester (II)

A solution of solid racemic hexahydro-cyclopenta[c]pyrrole-1,2-dicarboxylic acid 2-tert-butyl ester **(II),** obtained according to Example 3 or 4 (1.0 g, 3.92 mmol) in ethyl acetate (2.5 mL), is added to a solution of (S)-1,2,3,4-tetrahydronaphthylamine (0.317 g, 2.16 mmol) in ethyl acetate (1.7 mL) heated to 50°C under inert atmosphere. After 2 hours the reaction mixture is left to cool at room temperature; the solid is then filtered and washed with ethyl acetate to give the desired product (0.77 g, 48%, diastereomeric ratio 98.3/1.7 according to HPLC analysis).

¹H-NMR (CDCl₃): 7.41 (m, 1 H), 7.18 (m, 2 H), 7.07 (m, 1 H), 4.96 (bs, 4 H), 4.18 (m, 1 H), 3.95 (m, 1 H), 3.62 (m, 1 H), 3.18-3.05 (m, 2 H), 2.85-2.58 (m, 4 H), 2.10-1.63 (m, 7 H), 1-6-1.38 (m, 11 H).

### Example 6: Preparation of (1S,3aR,6aS) hexahydro-cyclo-penta[c]pyrrole-1,2-dicarboxylic acid 2-tert-butyl ester (II) wherein R is H and P is Boc

Solid (S)-1,2,3,4-tetrahydro-1-naphthylammonium **(II)** (1S,3aR,6aS) hexahydro-cyclopenta[c]pyrrole-1,2-dicarboxylate 2-tert-butyl ester, obtained according to Example 5 (0.77 g, 1.91 mmol), is suspended in ethyl acetate (25 mL), and the mixture is treated with a 1.2 M solution of HCl (50 mL) at room temperature. The resulting mixture is stirred for 15 minutes, and the phases are then separated. The product is extracted from the aqueous phase with ethyl acetate (3x20 mL). The combined organic phases are washed with water and a saturated solution of NaCl, dried on Na₂SO₄, filtered and concentrated at low pressure. Heptane (2 mL) is added to the residue thus obtained, and the mixture is left under stirring for 1 hour. The solid is filtered and washed with heptane to give chemically pure (1S,3aR,6aS) hexahydro-cyclopenta[c]pyrrole-1,2-dicarboxylic acid 2-tert-butyl ester **(II)** in enantiomerically enriched form (0.47 g, 96%), enantiomeric ratio 98.3/1.7), as a white crystalline solid.

### Example 7: Synthesis of racemic hexahydro-cyclopenta[c]pyrrole-1-carboxylic acid ethyl ester hydrochloride salt of formula (II), wherein P is H and R is ethyl

Hexahydro-cyclopenta[c]pyrrole-1,2-dicarboxylic acid 2-tert-butyl ester of formula **(II),** prepared according to Example 3 or 4 (10 g, 39.1 mmol), is treated with a saturated solution of gaseous HCl in ethanol (100 mL), and the resulting solution is maintained under stirring at the reflux temperature of the mixture for 24 hours. The end-of-reaction mixture is then concentrated at low pressure, and the residue is taken up with ethyl acetate (100 mL). The solid obtained is filtered and recrystallised from ethyl acetate to obtain the hexahydro-cyclopenta[c]pyrrole-1-carboxylic acid ethyl ester hydrochloride of formula **(II)** (5.6 g) with a potentiometric assay exceeding 96%.

### Example 8: Enzymatic preparation of (1S,3aR,6aS) hexahydro-cyclopenta[c]pyrrole-1,2-dicarboxylic acid 2-tert-butyl ester ethyl ester of formula (II), wherein R is ethyl and P is Boc

The hydrochloride salt of hexahydro-cyclopenta[c] pyrrole-1-carboxylic acid ethyl ester (100 mg, 0.62 mmol), prepared as in Example 7, is dissolved in 5 mL of 10 mM phosphate buffer at pH 7.0. The resulting solution is treated with 500 mg of lipase obtained from *Aspergillus niger.* The solution is placed under stirring at 130 rpm (rate per minute) at the temperature of 30°C. The reaction is stopped after 24 hours and treated with 2M NaOH (0.35 mL), ethyl acetate (8 mL) and di-tert-butyl dicarbonate (100 mg). The biphasic mixture is maintained under stirring at the temperature of about 20°C; the two phases are then separated, and the organic phase is dried on Na₂SO₄, filtered and concentrated at low pressure. Optically active hexahydro-cyclopenta[c]pyrrole-1,2-dicarboxylic acid 2-tertbutyl ester ethyl ester **(II),** wherein R is ethyl and P is Boc, is obtained with an enantiomeric purity exceeding 98:2, evaluated by HPLC on chiral stationary phase.

¹H-NMR (CDCl₃, T = 50°C): 4.17 (q, 2 H), 4.03 (m, 1 H), 3.68 (m, 1 H), 3.28 (m, 1 H), 2.62 (m, 2 H), 1.94 (m, 1 H); 1.9-1.7 (m, 2 H), 1.68-1.5 (m, 2 H), 1.48 (m, 1 H), 1.43 (s, 9 H), 1.28 (t, 3 H).

GC/MS (m/z): 281, 210, 182, 154, 110.

## Claims

1. A process for the preparation of a compound of formula **(II),** as a racemic mixture or a single enantiomer or a salt thereof 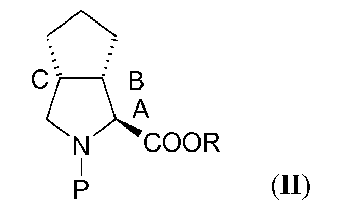 wherein R is H, an optionally substituted C₁-C₁₂ alkyl, an optionally substituted C₃-C₁₀ cycloalkyl, or an optionally substituted aryl or heteroaryl group; P is H or an amino protecting group comprising oxidising a compound of formula **(III)** as a stereoisomeric mixture or as a single enantiomer 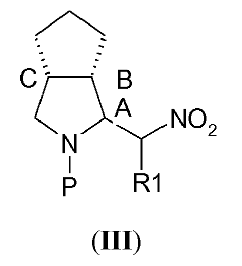 wherein P is as defined above and R1 is H or methyl and, if desired, optionally converting a compound **(II)** to another compound **(II)** or a salt thereof.

2. A process according to claim 1, wherein a compound of formula **(III)** wherein R1 is hydrogen is directly converted to a compound of formula **(II)** by treatment with an oxidising agent selected from the group consisting of potassium permanganate, sodium nitrite, potassium peroxymonosulphate, a mixture of potassium hydrogen sulphate and potassium peroxydisulphate and molecular oxygen in the presence of a copper-based catalyst.

3. A process according to claim 1, wherein a compound of formula **(III)** wherein R1 is hydrogen is oxidised in a first step to a compound of formula **(IV)** 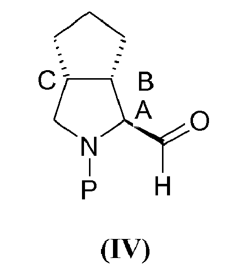 and then to a compound of formula **(II).**

4. A process according to claim 1, wherein a compound of formula **(III)** wherein R1 is methyl is oxidised to a compound of formula **(V)** 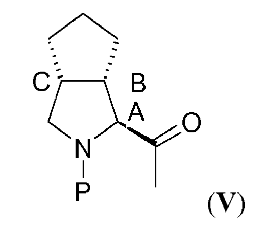 by treatment with an oxidising agent or by a Nef reaction, followed by conversion of compound **(V)** to compound **(II).**

5. A process according to claims 1-4, further comprising the resolution of the racemic mixture of a compound of formula **(II)** to give a compound of formula **(II)** in optically active form, wherein R and P are as defined in claim 1, by enantioselective enzymatic hydrolysis of the ester group of a compound of formula **(II)** wherein R, being as defined above is different from H, and P is as defined in claim 1.

6. A process according to claims 1-4, further comprising the resolution of a racemic mixture of a compound of formula **(II)** to give an enantiomerically pure compound **(II)** by formation of the diastereomeric salts thereof with a chiral amine.

7. A process according to claim 6, wherein the chiral amine is (S)-1,2,3,4-tetrahydro-1-naphthylamine, (S)- phenylethylamine or (S)- naphthylethylamine.

8. A process for the preparation of a compound of formula **(III),** as defined in claim 1, comprising adding a compound of formula **(VI)**
R1CH₂NO₂ **(VI)**
wherein R1 is as defined in claim 1, to a racemic compound of formula **(VII)** 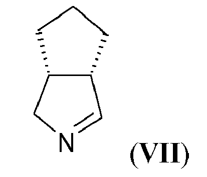 to obtain a compound of formula **(III)** 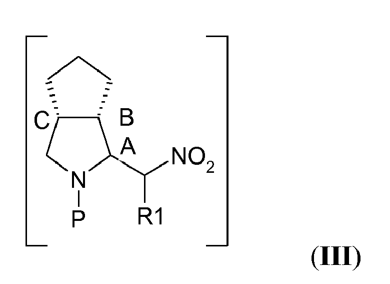 wherein R1 is as defined in claim 1 and P is H, and the subsequent protection of the amino group of said compound of formula **(III).**

9. A compound of formula **(III)** or **(IV)** or **(V)** 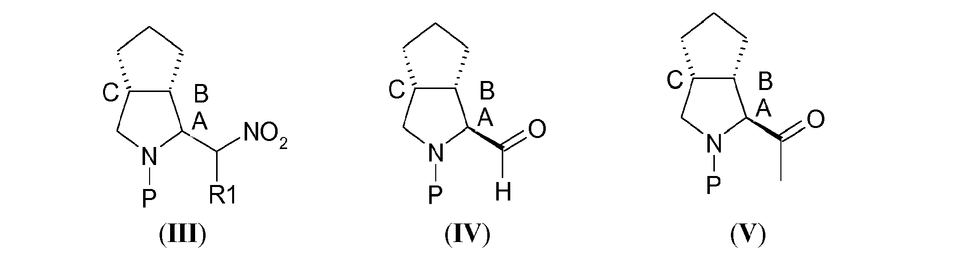 wherein P is H or an amino protecting group and R1 is hydrogen or methyl, wherein in a compound of formula **(IV)** P is not tert-butyloxycarbonyl (Boc).

10. A process according to claims 1-7, further comprising the conversion of a resulting compound of formula **(II)** to telaprevir of formula **(I)**

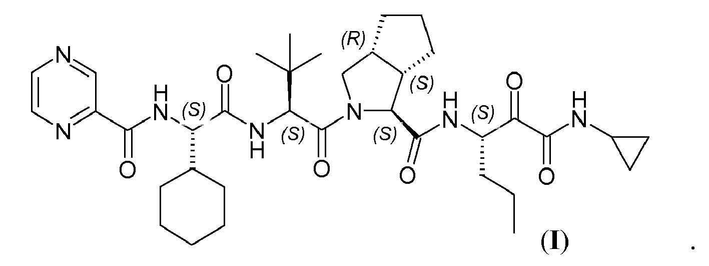

11. The use of a compound of formula **(III), (IV)** or **(V)** 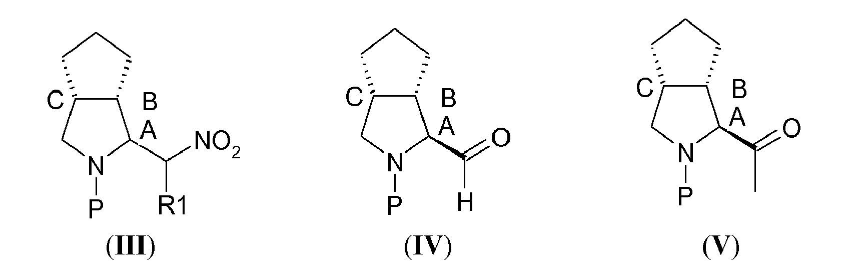 wherein P is H or an amino protecting group as an intermediate in a process for the preparation of telaprevir of formula **(I)**
